(19) Europäisches Patentamt
European Patent Office
Office européen des brevets

(11) Veröffentlichungsnummer: **0 312 734 B1**

# (12) EUROPÄISCHE PATENTSCHRIFT

(45) Veröffentlichungstag der Patentschrift: **02.01.92**

(51) Int. Cl.5: **C07D 487/08**, //(C07D487/08, 241:00,241:00)

(21) Anmeldenummer: **88113766.5**

(22) Anmeldetag: **24.08.88**

## (54) Verfahren zur Herstellung von 1,4-Diazabicyclo(2.2.2)-octan aus Piperazin.

(30) Priorität: **17.10.87 DE 3735214**

(43) Veröffentlichungstag der Anmeldung:
**26.04.89 Patentblatt 89/17**

(45) Bekanntmachung des Hinweises auf die Patenterteilung:
**02.01.92 Patentblatt 92/01**

(84) Benannte Vertragsstaaten:
**AT BE CH DE ES FR GB IT LI NL SE**

(56) Entgegenhaltungen:
**EP-A- 0 158 319**
**US-A- 3 772 293**
**US-A- 4 582 904**

(73) Patentinhaber: **HÜLS AKTIENGESELLSCHAFT**
**- RSP Patente / PB 15 - Postfach 13 20**
**W-4370 Marl 1(DE)**

(72) Erfinder: **Disteldorf, Josef, Dr.**
**Dahlbrede 47**
**W-4370 Marl(DE)**
Erfinder: **Finke, Norbert, Dr.**
**Untermarkstrasse 59 a**
**W-4600 Dortmund 30(DE)**
Erfinder: **Hübel, Werner, Dr.**
**Keplerstrasse 13**
**W-4350 Recklinghausen(DE)**

EP 0 312 734 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der Erfindung ist ein Verfahren zur Herstellung von 1,4-Diazabicyclo(2.2.2)-octan (auch Triethylendiamin genannt und im folgenden mit "TEDA" abgekürzt) aus Piperazin.

TEDA ist ein technisch bedeutsames Produkt; es wird vor allem als Katalysator bei der Herstellung von Polyurethanen benötigt.

In der Vergangenheit sind eine Vielzahl von Synthesen zur Herstellung von TEDA entwickelt worden, die sich einmal in der Art der Ausgangsprodukte und zum anderen in der Art der Katalysatoren unterscheiden. Verfahren, die von nichtcyclischen Ausgangsprodukten wie z. B. Ethylendiamin, Diethylentriamin, Ethylenoxid und Ethanolaminen ausgehen, haben den Nachteil, daß TEDA nur mit geringer Selektivität gebildet wird. Es kommt erschwerend hinzu, daß sich TEDA nur vergleichsweise aufwendig von den Nebenprodukten abtrennen läßt.

Die meisten technisch angewandten Verfahren zur Herstellung von TEDA gehen von Piperazinderivaten der allgemeinen Formel

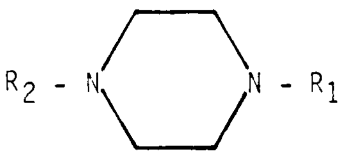

mit $R_1$ = $-CH_2-CH_2-XH$ mit X = O, NH
und $R_2$ = $R_1$, H
aus.

Bei diesem Verfahren werden entweder Phosphate oder Alumosilikate als Katalysatoren eingesetzt. So ist z. B. aus der DE-OS 14 45 418 ein Verfahren zur Herstellung von TEDA bekannt, bei dem man gasförmiges N-Hydroxyethylpiperazin bei einer Temperatur von 260 bis 427 °C in Gegenwart eines Verdünnungsmittels über einen festen, kieselsäurehaltigen Krack-Katalysator leitet.

Das Verfahren der DD-PS 206 896 besteht darin, Ammoniak und N-(Beta-aminoethyl)-piperazin und/oder N-(Beta-hydroxyethyl)-piperazin in Dampfform über einen porösen, $SiO_2$ und $Al_2O_3$ enthaltenden Katalysator zu leiten. Bereits dem Patentanspruch 1 ist zu entnehmen, daß Piperazin gebildet wird und zurückgeführt werden muß. Ein ähnliches Verfahren wird in der US-PS 3 120 526 beschrieben.

Man kann auch gemäß EP-PS 0 010 671 einen Katalysator verwenden, der zumindest 97 % $SiO_2$ enthält und eine innere Oberfläche nach BET von mehr als 30 $cm^2/g$ aufweist.

Schließlich gibt es auch Verfahren, bei denen als Katalysatoren Zeolithe eingesetzt wird. In der DE-OS 24 34 913 wird ein spezieller Zeolith der Formel

$$a(M_{2/n}O) \cdot (Al_2O_3) \cdot m(SiO_2)$$

eingesetzt, wobei a den Wert 1,0 ± 0,5 hat, M für ein Kation eines (Erd)Alkalimetalles, eines Elementes der Zinkfamilie, ein Proton oder ein Ammoniumion steht, n die Valenz des Kations angibt und m, das Verhältnis zwischen $SiO_2$ und $Al_2O_3$, eine Zahl von 2 bis 12 bedeutet. Bevorzugt werden Zeolithe vom Typ A, X und Y. Den Beispielen ist zu entnehmen, daß bei Verwendung von N-(Beta-hydroxyethyl)-piperazin mehr als 12 %, bei Verwendung N-(Beta-aminoethyl)-piperazin sogar mehr als 20 % Piperazin gebildet werden.

Auch bei dem Verfahren der JP-OS 75/58 096, bei dem ein Molekularsieb 4A eingesetzt wird, entsteht zu einem erheblichen Anteil Piperazin.

Die EP-OS 0 158 319 beschreibt ein Verfahren zur Herstellung von TEDA, bei dem man ein substituiertes Piperazin bei einer Temperatur von 250 bis 550 °C mit einer Raumgeschwindigkeit von 200 bis 2 500 $Stunden^{-1}$ über einen Zeolithen leitet, dessen $SiO_2/Al_2O_3$-Verhältnis größer als 20 ist. Zwar wird behauptet, TEDA werde mit einer Selektivität von mindestens 70 Mol.-% gebildet; tatsächlich ist den Beispielen zu entnehmen, daß auch Piperazin in einer Menge von 10 bis 50 % entsteht.

Es ist bei all diesen Verfahren als nachteilig anzusehen, daß die als Ausgangsprodukt verwendeten Piperazinderivate erst aus Piperazin oder niedermolekularen, cyclischen Verbindungen hergestellt werden müssen. Als doppelt nachteilig muß es angesehen werden, daß bei der Herstellung von TEDA zu einem erheblichen Anteil Piperazin gebildet wird. Es hat daher Bemühungen gegeben, TEDA direkt aus Piperazin herzustellen.

Nach dem Verfahren der US-PS 3 772 293 läßt sich TEDA durch Umsetzung von Piperazin mit

Ethylenoxid an einem aktivierten Kaolinkontakt in 59 %iger Ausbeute herstellen. Es ist davon auszugehen, daß bei diesem Verfahren intermediär N-(Beta-hydroxyethyl)-piperazin gebildet und dieses zu TEDA umgesetzt wird.

Aus dem Stand der Technik ist somit kein Verfahren zur Herstellung von TEDA bekannt, bei dem man von Piperazin als einzigem reaktiven Produkt ausgeht. Der Fachmann erwartet, daß eine direkte Umsetzung von Piperazin zu TEDA gar nicht möglich ist, da eine Ethyleneinheit in hochspezifischer Weise in das Piperazinmolekül eingebaut werden muß.

Es wurde jetzt überraschend gefunden, daß man Piperazin in Gegenwart von Zeolithen direkt zu TEDA umsetzen kann. Der verwendete Zeolith weist vorzugsweise die allgemeine Formel

$$(M^{I} \cdot M^{II}O_2) \cdot aM^{III}O_2 \cdot bH_2O$$

auf, wobei

- $M^{I}$ ein Äquivalent eines 1-einwertigen Alkalimetallkations, ein Proton, die Ammoniumgruppe oder ein halbes Äquivalent eines 2-wertigen Erdalkalimetallkations ist,
- $M^{II}$ ein Äquivalent eines der folgenden 3-wertigen Kationen ist $Al^{3+}$, $B^{3+}$, $Sb^{3+}$, $As^{3+}$, $Cr^{3+}$, $V^{3+}$, $Ga^{3+}$ und $Fe^{3+}$
- $M^{III}$ ein Äquivalent eines der folgenden 4-wertigen Kationen ist $Si^{4+}$, $Ge^{4+}$, $Ti^{4+}$ und $Zr^{4+}$
- a mindestentes den Wert 1 hat,
  und der Wert von
- b nach oben hin lediglich durch die Forderung begrenzt ist, daß eine Zeolith-Struktur aufrecht erhalten bleiben muß.

$M^{I}$ ist insbesondere ein Proton, $M^{II}$ $B^{3+}$ oder vorzugsweise $Al^{3+}$ und $M^{III}$ $Ge^{4}$ + oder vorzugsweise $Si^{4+}$. Es handelt sich vorzugsweise um einem Zeolithen mit Pentasil- und insbesondere ZSM 5-Struktur. Das Verfahren wird vorzugsweise so durchgeführt, daß man gasförmiges Piperazin oder Gemische aus TEDA und Piperazin bei einem Absolutdruck von 0,1 bis 10 bar über einen Zeolith-Festbettkatalysator leitet, eine Temperatur von 250 bis 550 °C, vorzugsweise 280 bis 380 °C, einstellt und die Katalysatorbelastung so einstellt, daß die LHSV 0,1 bis 10 Stunden$^{-1}$ beträgt.

Der genaue Mechanismus dieser Reaktion ist unklar, jedoch muß man auf Grund der gefundenen Stöchiometrie offenbar von einer Übertragung von Ethylengruppen unter Ammoniakabspaltung ausgehen. In der einschlägigen Literatur sind keine analogen Reaktionen bekannt.

Gegenstand der Erfindung ist ein Verfahren zur Herstellung von 1,4-Diazabicyclo(2,2,2)-octan, indem man cyclische Ausgangsverbindungen, gegebenenfalls unter Zusatz eines Verdünnungsmittels und gegebenenfalls eines Gases, mit Zeolithen bei 250 bis 550 °C und Drücken von 0,1-50 bar in Kontakt bringt, welches dadurch gekennzeichnet ist, daß man als einzige reaktive, cyclische Ausgangsverbindung Piperazin einsetzt und der Zeolith die allgemeine Formel

$$(M^{I} \cdot M^{II}O_2) \cdot aM^{III}O_2 \cdot bH_2O$$

aufweist, wobei

- $M^{I}$ ein Äquivalent eines einwertigen Alkalimetallkations, ein Proton, die Ammoniumgruppe oder ein halbes Äquivalent eines 2-wertigen Erdalkalimetallkations ist,
- $M^{II}$ ein Äquivalent eines der folgenden 3-wertigen Kationen ist $Al^{3+}$, $B^{3+}$, $Sb^{3+}$, $As^{3+}$, $Cr^{3+}$, $V^{3+}$, $Ga^{3+}$ und $Fe^{3+}$
- $M^{III}$ ein Äquivalent eines der folgenden 4-wertigen Kationen ist $Si^{4+}$, $Ge^{4+}$, $Ti^{4+}$ und $Zr^{4+}$
- a einen Wert zwischen 15 und 200 besitzt

und der Wert von

- b zwischen 0 und 8 liegt.

Mit dem erfindungsgemäßen Verfahren werden folgende Vorteile erzielt:

1. Das als Ausgangsprodukt verwendete Piperazin ist eine technisch leicht zugängliche, preisgünstige Verbindung, die beispielsweise als Nebenprodukt bei der Herstellung von Ethylendiamin anfällt.
2. Man erzielt eine hohe Selektivität. Das einzige, in nennenswerten Mengen auftretende Nebenprodukt ist unumgesetztes Piperazin.
3. Gemische aus nicht umgesetztem Piperazin und TEDA können erneut dem Katalysator zugeführt werden.

Grundsätzlich können bei dem erfindungsgemäßen Verfahren alle bekannten Zeolithe eingesetzt werden. Zeolithe und deren Herstellung werden beispielsweise in Ullmanns Encyclopädie der Technischen Chemie, 4. Auflage, Band 24, Seite 575 (1983) und Band 17, Seite 9 (1979) beschrieben.

Die Zeolithe weisen insbesondere die oben angegebene Formel auf. Vorzugsweise hat a einen Wert zwischen 15 und 200 und b einen Wert zwischen 0 und 8. Typischerweise steht $M^{II}$ für $Al^{3+}$ und $M^{III}$ für $Si^{4+}$. Ersetzt man eines dieser beiden Metalle im Sinne einer sogenannten isomorphen Substitution durch andere Metalle, wie sie oben angegeben sind, so erhält man Zeolithe, deren Kristallstruktur im wesentlichen unverändert ist (vgl . Chem.-Ing. Techn. 58, 946 - 959 und 969 - 971 (1986). Es ist auch möglich, sowohl Aluminium als auch Silizium zu ersetzen. Es besteht auch die Möglichkeit der teilweisen Substitution. Die'Zeolithe können auch mit Übergangsmetallen und/oder Seltenen Erden dotiert werden. Zur Erhöhung der Standfestigkeit der Zeolithen werden diese vielfach mit anderen Materialien wie z. B. Cellulosematerialien, Tonen, Metalloxiden oder Bindemitteln versetzt und erhalten so ihre Form. Bevorzugt sind Zeolithe, die eine Pentasil-Struktur aufweisen. Geeignete Zeolithe werden beispielsweise in der DE-OS 32 39 054 beschrieben. Insbesondere werden Zeolithe mit ZSM 5-Struktur verwendet, die in der obengenannten Monographie aufgeführt sind.

Es ist zweckmäßig, das Piperazin zusammen mit einem Verdünnungsmittel, wie z. B. Wasser, einem inerten Kohlenwasserstoff, der bei Raumtemperatur flüssig ist, und einem Gas, wie z. B. Stickstoff oder Ammoniak, dem Katalysator zuzuführen. Dabei geht man üblicherweise so vor, daß man Piperazin in Wasser löst und zusammen mit der gasförmigen Komponente in einer geeigneten Aufwärmvorrichtung auf eine Temperatur aufheizt, bei der Piperazin in gasförmigen Zustand vorliegt. Man kann das Piperazin auch in flüssigen Kohlenwasserstoffen lösen und entsprechend verfahren.

Der Druck ist keine kritische Größe des vorliegenden Verfahrens, obwohl dem Fachmann bekannt ist, daß der Umsatz mit steigendem Druck zunimmt. Im allgemeinen sollte der Druck zwischen 0,1 und 50 bar liegen. Das Verfahren besteht grundsätzlich darin, daß das Piperazin mit dem Zeolithen in Kontakt gebracht wird. Es ist üblich, gasförmiges Piperazin in einem Rohr über den fest angeordneten Zeolithen zu leiten. Die Katalysatorbelastung wird vorzugsweise so eingestellt, daß die LHSV (liquid hourly space velocity) 0,1 bis 10 $Stunden^{-1}$ beträgt (vgl. Ullmanns Encyklopädie der technischen Chemie, 4. Auflage, Band 13, Seite 550, Verlag Chemie, Weinheim, 1977).

Die Aufarbeitung des Reaktionsgemisches kann nach üblichen Methoden erfolgen. Es hat sich als vorteilhaft erwiesen, alle produktführenden Leitungen, Pumpen und Abscheider auf eine Temperatur von 40 bis 80 °C aufzuheizen. Eine besonders zweckmäßige Form besteht darin, das Reaktionsgemisch aufzufangen und destillativ aufzutrennen. Sofern gewünscht, kann TEDA zur Verbesserung seiner Reinheit aus geeigneten Lösemitteln umkristallisiert werden. Ein besonderer Vorteil des Verfahrens besteht darin, daß man Zwischenfraktionen, die sowohl TEDA als auch Piperazin enthalten, erneut dem Katalysator zuführen kann. Der Versuch 6 und Vergleichsversuch A zeigen, daß TEDA unter diesen Umständen nur unwesentlich geschädigt wird.

Beschreibung der Versuchsapparatur

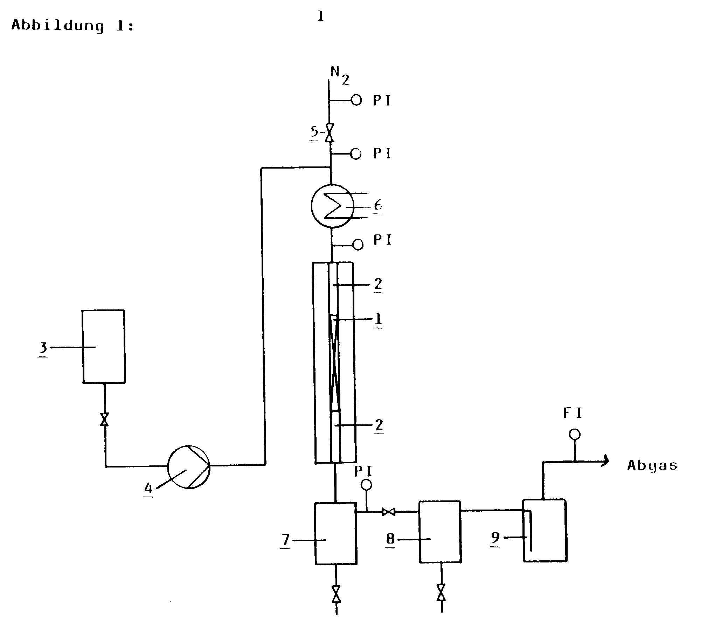

Die Versuche werden in einem Festbett-Rohrreaktor aus Edelstahl (Länge 1350 mm, Durchmesser 22 mm) mit Sandwirbelbettbeheizung und einem innenliegenden Temperaturmeßrohr (Durchmesser 6 mm) durchgeführt. In Abbildung 1 ist der Versuchsaufbau schematisch dargestellt. In der Mitte des Reaktors befindet sich eine 350 mm lange Kontaktzone (1), die mit 120 ml Katalysator gefüllt ist und an die sich oben und unten zwei 500 mm lange Füllkörperschichten (2) anschließen. Das Einsatzprodukt wird aus der kalibrierten Vorlage (3) über eine Membranpumpe (4) dosiert und zusammen mit einem Stickstoffstrom, der über ein Reduzierventil (5) geregelt wird, in den Wärmetauscher (6) auf 200 °C aufgeheizt und von oben in den Reaktor geleitet. Die Reaktionsprodukte werden in zwei hinter einander geschalteten Abscheidern (7 und 8) und einer mit Wasser gefüllten Waschflasche (9) aufgefangen; der Abgasstrom wird über ein Ventil und eine Gasuhr (FI) geregelt. Die Aufarbeitung der Reaktionsprodukte erfolgt durch Destillation in einer Füllkörperkolonne (Länge 1700 mm, Durchmesser 30 mm, gefüllt mit V4A-Maschendrahtringen 4 x 4 mm) mit thermostatisierbarem Kolonnenkopf. Der Destillationsschnitt mit einer Siedetemperatur von 140 - 160 % besteht im wesentlichen aus Piperazin mit geringen Mengen TEDA und kann zurückgeführt werden. Die Fraktion von 160 bis 180 °C enthält die Hauptmenge TEDA neben geringen Mengen Piperazin. Durch eine erneute Feindestillation in der gleichen Apparatur können die beiden Komponenten getrennt werden. Durch Umkristallisation aus Ethylacetat erhält man farbloses TEDA in einer Reinheit von mehr als 99,5 %.

Als kristalline Zeolithe vom Pentasiltyp wurden solche verwendet, deren Herstellung in der DE-OS 32 39 054 beschrieben ist.

In den folgenden Beispielen Nr. 1, 2, 3, 4, 6 und 7 ist der mit A bezeichnete Katalysator ein Zeolith in der H-Form nach Beispiel 1 der DE-OS 32 39 054 (molares Verhältnis $SiO_2/Al_2O_3$ = 30,8), der zur Konfektionierung mit 40 Gew.-% eines inerten Bindemitels ausgeformt war. Der mit B bezeichnete Katalysator in Beispiel 5 entspricht dem Zeolithen nach Beispiel 4 der DE-OS 32 39 054 (molares Verhältnis

$SiO_2/Al_2O_3$ = 90,0), ebenfalls mit 40 Gew.-% eines inerten Bindemittels konfektioniert.

Beispiele

In der Tabelle sind die Ergebnisse der Beispiele 1 bis 6 und des Vergleichsbeispiels A aufgeführt. In den Beispielen 1 bis 5 wird als Einsatzprodukt eine 40 %ige wäßrige Piperazinlösung verwendet. Mit dem Katalysator A wurde der Einfluß der Reaktionstemperatur, des Druckes und der Katalysatorbelastung auf Umsatz und Selektivität untersucht (Beispiele 1 bis 4). Der Einfluß des Katalysators geht aus dem Vergleich von Beispiel 5 mit Beispiel 1 hervor. In Beispiel 6 wurde ein Reaktionsprodukt mit 58,0 % Wasser, 16,8 % TEDA und 23,3 % Piperazin erneut dem Katalysator zugeführt. Bei nur geringfügiger Einbuße der Selektivität kann der Umsatz beträchtlich gesteigert werden. Das Einsatzprodukt in Vergleichsbeispiel A ist eine 40 %ige wäßrige TEDA-Lösung. Man erkennt, daß TEDA unter den Reaktionsbedingungen nahezu stabil ist.

Tabelle

| | | | | | | Reaktionsprodukte[1] | | | | | | | |
|---|---|---|---|---|---|---|---|---|---|---|---|---|---|
| Beisp. Nr. | Kat. | Temp. ˚C | Einsatzmenge ml/h | Abgas[3] l/h | Druck (abs.) bar | Wasser % | PIP % | TEDA % | EtP % | DPE % | DETA % | Umsatz % | Selektivität2) % |
| 1 | A | 340 | 50 | 100 | 3 | 59,60 | 22,22 | 16,56 | 0,91 | 0,12 | 0,06 | 45 | 91 |
| 2 | A | 310 | 100 | 100 | 3 | 58,75 | 29,66 | 10,31 | 0,35 | 0,73 | 0,03 | 26 | 89 |
| 3 | A | 310 | 100 | 100 | 2 | 57,91 | 32,98 | 8,35 | 0,21 | 0,45 | 0 | 18 | 92 |
| 4 | A | 340 | 50 | 200 | 3 | 55,70 | 25,46 | 17,25 | 0,86 | 0,17 | 0,08 | 36 | 92 |
| 5 | B | 340 | 50 | 100 | 3 | 58,10 | 22,81 | 17,43 | 0,80 | 0,15 | 0,05 | 43 | 91 |
| 6 | A | 340 | 50 | 100 | 3 | 55,70 | 13,61 | 27,10 | 1,91 | 0,20 | 0,10 | 66 | 88 |
| A | A | 340 | 50 | 100 | 3 | 60,05 | 0,50 | 39,05 | 0,23 | 0 | 0 | 0 | 0 |

Verwendete Abkürzungen:

EtP = 1-Ethylpiperazin

DETA = Diethylentriamin

DPE = 1,2-Dipiperazinoethan

PIP = Piperazin

[1] Die analytische Bestimmung der Reaktionsprodukte erfolgte nach Abzug von Ammoniak. Der Wasseranteil wurde nach der Karl-Fischer-Wasserbestimmung ermittelt. Der Anteil der Amine wurde gaschromatographisch ermittelt.

[2] Als Selektivität wird im Rahmen dieser Erfindungsmeldung der Massenanteil von TEDA in den Reaktionsprodukten nach Abzug von Ammoniak und Wasser, bezogen auf umgesetztes Piperazin, angegeben.

[3] Das Abgas besteht im wesentlichen aus dem zugeführten Schleppgas (Stickstoff) und einen geringen Anteil von Ammoniak.

## Patentansprüche

1. Verfahren zur Herstellung von 1,4-Diazabicyclo(2.2.2)-octan, indem man cyclische Ausgangsverbindungen, gegebenenfalls unter Zusatz eines Verdünnungsmittels und gegebenenfalls eines Gases, mit Zeolithen bei 250 bis 550 °C und Drücken von 0,1-50 bar in Kontakt bringt,
dadurch gekennzeichnet,
daß man als einzige reaktive, cyclische Ausgangsverbindung Piperazin einsetzt und der Zeolith die allgemeine Formel

$$(M^{I} \cdot M^{II}O_2) \cdot aM^{III}O_2 \cdot bH_2O$$

aufweist, wobei
$M^{I}$ ein Äquivalent eines einwertigen Alkalimetallkations, ein Proton, die Ammoniumgruppe oder ein halbes Äquivalent eines 2-wertigen Erdalkalimetallkations ist,
$M^{II}$ ein Äquivalent eines der folgenden 3-wertigen Kationen ist $Al^{3+}$, $B^{3+}$, $Sb^{3+}$, $As^{3+}$, $Cr^{3+}$, $V^{3+}$, $Ga^{3+}$ und $Fe^{3+}$
$M^{III}$ ein Äquivalent eines der folgenden 4-wertigen Kationen ist $Si^{4+}$, $Ge^{4+}$, $Ti^{4+}$ und $Zr^{4+}$
a einen Wert zwischen 15 und 200 besitzt
und der Wert von
b zwischen 0 und 8 liegt.

2. Verfahren gemäß Anspruch 1,
dadurch gekennzeichnet,
daß der eingesetzte Zeolith eine Pentasil-Struktur aufweist.

3. Verfahren gemäß Anspruch 2,
dadurch gekennzeichnet,
daß der eingesetzte Zeolith eine ZSM 5-Struktur aufweist.

4. Verfahren gemäß Anspruch 1,
dadurch gekennzeichnet,
daß
$M^{I}$ ein Proton
$M^{II}$ $B^{3+}$ oder vorzugsweise $Al^{3+}$
und
$M^{III}$ $Ge^{4+}$ oder vorzugsweise $Si^{4+}$ ist.

5. Verfahren gemäß den Ansprüchen 1 bis 4,
dadurch gekennzeichnet,
daß man gasförmiges Piperazin über einen Festbettkatalysator bei einem Absolutdruck von 0,1 bis 10 bar leitet.

6. Verfahren gemäß Anspruch 5,
dadurch gekennzeichnet,
daß man eine Katalysatorbelastung (LHSV) von 0,1 bis 10 pro Stunde einstellt.

7. Verfahren gemäß den Ansprüchen 1 bis 6,
dadurch gekennzeichnet,
daß man - anstelle von Piperazin - Gemische aus 1,4-Diazabicyclo-(2.2.2)-octanund Piperazin als cyclische Ausgangsverbindung einsetzt.

## Claims

1. A process for the preparation of 1,4-diazabicyclo(2.2.2)-octane, wherein cyclic starting compounds are brought into contact with zeolites at 250 to 550 °C and pressures of 0.1-50 bar, if necessary with the addition of a diluting agent and if necessary a gas,

characterized in that
the single reactive cyclic starting compound used is piperazine and the zeolite has the general Formula

$(M^{I} \cdot M^{II}O_2) \cdot aM^{III}O_2 \cdot bH_2O$

where,

- $M^{I}$ is an equivalent of a monovalent alkali-metal cation, a proton, the ammonium group or a semi-equivalent of a bivalent alkaline-earth metal cation,
- $M^{II}$ is an equivalent of one of the following trivalent cations: $Al^{3+}$, $B^{3+}$, $Sb^{3+}$, $As^{3+}$, $Cr^{3+}$, $V^{3+}$, $Ga^{3+}$ and $Fe^{3+}$
- $M^{III}$ is an equivalent of one of the following quadrivalent cations: $Si^{4+}$, $Ge^{4+}$, $Ti^{4+}$ and $Zr^{4+}$
- a has a value between 15 and 200

and the value of

- b is between 0 and 8.

**2.** A process according to claim 1, characterized in that the zeolite used has a pentasil structure.

**3.** A process according to claim 2, characterized in that the zeolite used has a ZSM 5 structure.

**4.** A process according to claim 1, characterized in that

- $M^{I}$ is a proton
- $M^{II}$ is $B^{3+}$ or preferably $Al^{3+}$, and
- $M^{III}$ is $Ge^{4+}$ or preferably $Si^{4+}$.

**5.** A process according to claims 1 to 4, characterized in that gaseous piperazine is passed over a solid bed catalyst at an absolute pressure of 0.1 to 10 bar.

**6.** A process according to claim 5, characterized in that a catalyst loading (LHSV) is adjusted from 0.1 to 10 per hour.

**7.** A process according to claims 1 to 6, characterized in that instead of piperazine, mixtures of 1,4-diazabicyclo-(2.2.2)-octane and piperazine are used as the cyclic starting compound.

**Revendications**

**1.** Procédé pour la fabrication du 1,4-diazabicyclo (2.2.2)-octane dans lequel on met en contact des composés de départ cycliques, éventuellement avec addition d'un agent de dilution, et éventuellement d'un gaz, avec des zéolites à une température de 250 à 550 °C et à des pressions allant de 0,1 à 50 bars, caractérisé en ce que l'on met en oeuvre à titre de produit de départ unique, réactif cyclique de la pipérazine et que la zéolite possède la formule générale :

$(M^{I} \cdot M^{II}O_2 \cdot aM^{III}O_2 \cdot bH_2O$

dans laquelle
$M^{I}$ est un équivalent d'un cation de métal alcalin monovalent, un proton, le groupe ammonium ou un demiéquivalent d'un cation de métal alcalino-terreux divalent.
$M^{II}$ est un équivalent d'un des cations trivalents suivants : $Al^{3+}$, $B^{3+}$, $Sb^{3+}$, $As^{3+}$, $Cr^{3+}$, $V^{3+}$, $Ga^{3+}$ et $Fe^{3+}$.
$M^{III}$ est un équivalent d'un des cations tétravalents suivants : $Si^{4+}$, $Ge^{4+}$, $Ti^{4+}$ et $Zr^{4+}$
a possède une valeur entre 15 et 200,
et la valeur de b se situe entre 0 et 8.

**2.** Procédé selon la revendications 1, caractérisé en ce que la zéolite mise en jeu possède une structure de pentasil.

**3.** Procédé selon la revendication 2, caractérisé en ce que la zéolite mise en jeu possède une structure ZSM 5.

**4.** Procédé selon la revendication 1, caractérisé en ce que :
$M^{I}$ est un proton,
$M^{II}$ est $B^{3+}$ et de préférence $Al^{3+}$ et
$M^{III}$ est $Ge^{4+}$ ou de préférence $Si^{4+}$.

**5.** Procédé selon les revendications 1 à 4, caractérisé en ce que l'on amène de la pipérazine gazeuse sur un catalyseur à lit solide, à une pression absolue de 0,1 à 10 bars.

**6.** Procédé selon la revendication 5, caractérisé en ce qu'on ajuste une charge du catalyseur (LHSV) de 0,1 à 10 par heure -1.

**7.** Procédé selon les revendications 1 à 6, caractérisé en ce que l'on met en oeuvre à la place de pipérazine, des mélanges de 1,4-diazobicyclo-(2,2,2)-octane et de pipérazine en tant que produit de départ cyclique.

Abbildung 1:

1